(19) Europäisches Patentamt
European Patent Office
Office européen des brevets

(11) **EP 2 856 119 B1**

(12) **EUROPEAN PATENT SPECIFICATION**

(45) Date of publication and mention
of the grant of the patent:
**08.01.2020 Bulletin 2020/02**

(21) Application number: **13800506.1**

(22) Date of filing: **19.02.2013**

(51) Int Cl.:
*G01N 21/78* (2006.01)    *G01N 21/77* (2006.01)
*G01N 31/22* (2006.01)

(86) International application number:
**PCT/US2013/026701**

(87) International publication number:
**WO 2013/184182 (12.12.2013 Gazette 2013/50)**

(54) **OPTICAL SENSOR FOR DETERMINING QUATERNARY AMMONIUM COMPOUND CONCENTRATION**

OPTISCHER SENSOR ZUR BESTIMMUNG DER KONZENTRATION EINER QUATERNÄREN AMMONIUMVERBINDUNG

CAPTEUR OPTIQUE PERMETTANT DE DÉTERMINER LA CONCENTRATION EN UN COMPOSÉ D'AMMONIUM QUATERNAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2012 US 201261655719 P**

(43) Date of publication of application:
**08.04.2015 Bulletin 2015/15**

(73) Proprietor: **Ecolab USA Inc.**
**St. Paul, MN 55102 (US)**

(72) Inventors:
• **KRAUS, Paul R.**
**Apple Valley, Minnesota 55124 (US)**
• **TISCHLER, Sherri**
**Inver Grove Heights, Minnesota 55076 (US)**
• **SMITH, Kim R.**
**Woodbury, Minnesota 55125 (US)**

(74) Representative: **Godemeyer Blum Lenze**
**Patentanwälte**
**Partnerschaft mbB - werkpatent**
**An den Gärten 7**
**51491 Overath (DE)**

(56) References cited:
WO-A1-00/61200          DE-A1- 3 614 835
US-A- 2 471 861          US-A- 2 979 468
US-A- 5 164 796          US-A1- 2005 104 035
US-A1- 2005 249 791      US-A1- 2006 293 205

US-A1- 2007 188 759      US-A1- 2008 219 552
US-A1- 2009 262 351      US-A1- 2009 284 732
US-A1- 2010 247 371      US-A1- 2010 247 371

• **BAUMES L A ET AL: "First colorimetric sensor array for the identification of quaternary ammonium salts", TETRAHEDRON LETTERS, PERGAMON, GB, vol. 50, no. 50, 16 December 2009 (2009-12-16), pages 7001-7004, XP026721347, ISSN: 0040-4039, DOI: 10.1016/J.TETLET.2009.09.165 [retrieved on 2009-10-02]**
• **J B LOWRY: "Direct Spectrophotometric Assay of Quaternary Ammonium Compounds Using Bromthymol Blue", JOURNAL OF PHARMACEUTICAL SCIENCES, vol. 68, no. 1, 1 January 1976 (1976-01-01), pages 110-111, XP055238086,**
• **Anonymous: "(Annex 3) List of dyes that generate specific aromatic amines (Informative)", , 1 October 2015 (2015-10-01), XP055600381, Retrieved from the Internet: URL:http://www.jtf-net.com/english/data/15 0901Guideline_annex3_en.pdf [retrieved on 2019-06-28]**

EP 2 856 119 B1

**(Cont. next page)**

- Anonymous: "TRADE AND ENVIRONMENT AND INTERNATIONAL COMPETITIVENESS IN THE MEDITERRANEAN REGION: SELECTED CASE STUDIES", MEDPOLICIES INITIATIVE MEDITERRANEAN ENVIRONMENTAL TECHNICAL ASSISTANCE PROGRAM (METAP), 1 January 2002 (2002-01-01), page 70, XP055600392, Retrieved from the Internet: URL:https://www.academia.edu/9590620/THE_EFFECT_OF_ENVIRONMENTAL_LEGISLATION_ON_FERTILIZER_EXPORTS_THE_CASE_OF_JORDAN1 [retrieved on 2019-06-28]

**Description**

**Background**

Maintaining adequate levels of antimicrobial concentration is necessary to ensure that an antimicrobial solution is effective at reducing microorganisms. This is important in industries that are sensitive to microorganisms like the food service or healthcare industries where microorganisms can cause food spoilage, illness, or infection. Frequently replacing an antimicrobial solution is one way to maintain the antimicrobial concentration. But, this may result in wasting active antimicrobial solution.

US 2010/0247371 A1 discloses a nonwoven wipe coated with a reversible color-changing ink formulation. During use, the wipe is impregnated with a quaternary ammonium compound-based sanitizer. When the level of free quaternary ammonium compound falls below a threshold level, the color-changing ink formulation changes from a first color to a second color, indicating the need to recharge the wipe. When the wipe is recharged with sanitizer solution, the color-changing ink formulation changes back to the first color.

There is a need for improved, precise methods of monitoring antimicrobial concentrations.

**Summary**

Surprisingly, it has been discovered that optical sensors can be used to monitor and report the concentration of antimicrobials and in particular quaternary ammonium compounds. Accordingly, the present disclosure generally relates to the use of an optical sensor to measure light intensity as a function of the wavelength of a dye that reacts with a quaternary ammonium compound. This measurement serves as an indicator for the concentration of a quaternary ammonium compound, or quat.

Accordingly, in some aspects, the disclosure relates to a system for determining the concentration of a quaternary ammonium compound where the system includes a dye. The dye has a first wavelength at a first concentration of a quaternary ammonium compound and a second wavelength at a second concentration of a quaternary ammonium compound. The system also includes a sensor capable of measuring the light intensity of the dye as a function of the wavelength of the dye and converting that measurement to a digital output.

In some aspects, the disclosure relates to a method of monitoring the concentration of a quaternary ammonium compound by contacting an antimicrobial solution having a quaternary ammonium compound concentration greater than a certain threshold with a dye. The method includes a dye where the dye has a first wavelength if the concentration of the quaternary ammonium compound is above the threshold and a second wavelength if the concentration of the quaternary ammonium compound is below the threshold. The method further includes measuring the wavelength of a dye with a sensor that measures the light intensity of the dye and converts the light intensity measurement to a digital output that corresponds to the concentration of quaternary ammonium compound. The method further includes using the quaternary ammonium solution for a period of time. The method includes adding quaternary ammonium solution in response to the dye changing from a first wavelength to a second wavelength as the quaternary ammonium compound concentration drops below the threshold.

In some aspects, the disclosure relates to a method of monitoring the concentration of a quaternary ammonium compound in a container by placing a sensor into the container. The sensor measures the color of a dye that is in contact with a quaternary ammonium compound antimicrobial solution in the container. The method includes measuring light intensity as a function of the wavelength of a dye with the sensor. The dye has a first wavelength if the concentration of the quaternary ammonium compound is above 100 ppm and a second wavelength if the concentration of the quaternary ammonium compound is below 100 ppm. The method includes converting the sensor's light intensity measurement to a digital output that corresponds to the concentration of quaternary ammonium compound. The method further includes using the quaternary ammonium solution for a period of time and adding quaternary ammonium solution in response to the dye changing from a first wavelength to a second wavelength if the concentration drops below 100 ppm of the quaternary ammonium compound.

The invention is defined in claim 1. Embodiments are defined in the dependent claims, and will be apparent to those skilled in the art and others in view of the following detailed description. It is understood, however, that this summary, and the detailed description illustrate only some examples, and are not intended to be limiting to the claimed invention. Examples described in the following do not necessarily fall within the scope of the claimed invention.

**Brief Description of the Drawings**

**Figure 1** shows color readings for the RGB channels of a color sensor showing the quat concentration dependent responses.

**Figure 2** shows the combined color readings for the RGB channels of a color sensor showing the quat concentration dependent responses.

## Detailed Description

The present disclosure generally relates to using an optical sensor that measures light intensity as a function of the wavelength of a dye, which in the presence of quat has a first wavelength if the concentration of the quaternary ammonium compound is above a threshold and a second wavelength if the concentration of the quaternary ammonium compound is below a threshold. Because the wavelength corresponds to the quaternary ammonium compound concentration, the sensor and dye can serve as a real time indicator for quat concentration. An optical sensor measuring the dye signals when the concentration of the quat changes. The dye is preferably pH stable, meaning that the wavelength change of the dye associated with the change in quat concentration is independent of the pH of the quat solution. Put another way, the dye preferably performs the same if the antimicrobial solution is at an acidic, neutral, or basic pH. This is in contrast to pH indicator dyes that are specifically designed to change color as the pH changes. In some examples, the dye is reversible in that once it changes from a first wavelength to a second wavelength, it can also change from the second wavelength back to the first wavelength. This is in contrast to indicator dyes, such as those on quaternary ammonium test strips that do not change back to their original color once they have changed color.

In some examples, the change may be multicolored or over multiple wavelengths where the dye changes from a first wavelength to a second wavelength as the concentration nears a first threshold (such as a warning threshold), and then changes from the second wavelength to the third wavelength once the concentration reaches a second threshold (such as the point where the solution is no longer an effective antimicrobial solution). In some examples, the wavelength change is arbitrary and not linked to the effectiveness of the solution. In such examples, the change serves as a reminder to replace the antimicrobial solution before the solution stops being effective. In some examples, the change can be triggered by a time lapse and not the quaternary ammonium concentration.

In some examples, the "change" in dye wavelength includes a change from a first color to a second color, or from no color to color, or color to no color. A visual color change may be desirable in addition to the output from the sensor. In some examples, the "color change" can include words or symbols appearing where words or symbols were not previously there or were a different color. The word could be part of an article or part of the output from the sensor. Alternatively, a container may go from clear or colorless to colored, or white to colored or vice versa (colored to clear/colorless or colored to white) for the "color change."

In some examples, the sensor numerically describes the concentration of quat, such as in parts per million or percentages. In some examples, the sensor's output reveals a word which calls for the introduction of additional quat. In some examples, the sensor provides a digital output that would reveal a symbol, which would call for the introduction of additional quat. In other examples, the sensor provides an alert as an output (visual, audio, vibration etc.), which calls for the introduction of additional quat. The sensor signal would occur when the level of quat drops below a predetermined critical level. The output may include words, phrases, instructions, or graphics for the user to check the concentration of the antimicrobial solution, such as: "CHANGE", "REPLACE", "QUAT", "IT IS TIME TO CHANGE THE SOLUTION", "NO QUAT" and the like. Alternatively, the digital output can also include a word or phrase disappearing such as: "QUAT", "ANTIMICROBIAL" and the like.

In some examples, the wavelength change occurs in the visible range and shows up as a color change. These examples allow a user to identify the color change visually and numerically or digitally. In some examples, the "color change" or "wavelength change" may occur outside of the visible range of colors and may be detected by an optical sensor instead of or in addition to visually. Additionally, the "color change" can be such that, even in the visible range, the change in color is too slight to perceive visually, but can nevertheless be quantified by the sensor. In such examples where the color change is not perceptible to the eye or occurs outside of the visible range, the optical sensor can be used to measure the change.

Exemplary optical sensors include color (RGB) sensors that measure the color or wavelength of a substrate such as a test strip, coupon, probe, tag, container, article, or the like. One example of a commercially available sensor is the TCS230LM programmable color light-to-frequency converter from Texas Advanced Optoelectronic Systems (Piano, Texas). Other examples include the CZ-V20 Series or CZ Series RGB sensors from Keyence America, the S9702 RGB sensor from Hamamatsu Japan, the MAX44006 and MAX44008 RGB sensors from Maxim, the D800 or D800E image sensor from Nikon, or the ColorMax-1000 Discrete, ColorMax-1000 Hex, ColorMax-1000 RGB, or ColorMax-1000 Flex sensors from EMX Industries, Inc.

An integrated light-to-frequency converter provides RGB color sensing and is performed by a photodiode grid consisting of 16 groups of 4 elements each. Each group consists of a red sensor, a green sensor, a blue sensor, and a clear sensor with no filter. The sensor can detect the light loss at the interface of a dye due to interaction of the dye with quat. The digital output for each color is a square wave whose frequency is directly proportional to the intensity of the selected color. The color information is input directly to a processor by sequentially selecting each color channel, then counting pulses or timing the period to obtain a value. The use of light-to-frequency eliminates circuitry since transimpedance amplifiers and analog-to-digital converters are no longer needed. Furthermore, the sensor may be located remotely from the processor with no loss of noise immunity.

In some examples, an optical sensor can be designed as a handheld pen-sized sensor for measuring quat concentration in buckets, sinks or the like. Such a sensor could be found in a "pen"- or "flashlight"-like article that a user flashes at the article. Another exemplary sensor is a pen that has the dye component integrated into the pen that also contains a light source and the sensor. In some examples, the sensor may be integral with a container such that the sensor provides a constant or periodic reading of the quaternary ammonium compound concentration in the container.

The sensor may optionally include a transmitter and receiver such as in a wireless device that would be able to identify a remote solution as being near or below a threshold and send a signal to a location (such as a computer terminal or cloud network) that would alert a user that the solution needs to be adjusted or replaced.

In some examples, an optical sensor may be designed as a single-use test surface where it is placed in a solution to read changes in light intensity as a function of wavelength and therefore the quat concentration. In some examples, the wavelength changes as the concentration passes a certain threshold. In some examples, the threshold is the point where the antimicrobial solution goes from being an effective antimicrobial solution to not being an effective antimicrobial solution. For example, for some applications, the antimicrobial solution preferably includes at least about 100, 150, or 200 ppm of quaternary ammonium compound for the overall solution to be effective. For other applications, the antimicrobial solution preferably includes at least about 600, 800, 1000, or 2000 ppm of quaternary ammonium compound for the overall solution to be effective. Therefore, in some examples, the disclosed dye changes wavelength as the concentration drops under 100, 150, 200, 600, 800, 1000, or 2000 ppm quaternary ammonium compound. In some examples, the wavelength changes before the quaternary ammonium compound drops under the threshold to signal that the concentration of quaternary ammonium compound is getting close to being low. For example, it may be desirable for the wavelength change to happen at least 100 ppm before the concentration of quaternary ammonium compound drops under the threshold, i.e., 200, 250, 300, 700, 900, 1100, or 2100 ppm if using the above stated thresholds. In some examples, it may be desirable for the wavelength change to be gradual as the concentration nears the threshold and complete once the concentration reaches threshold quaternary ammonium compound concentration.

In some examples, the dye may be immobilized on a clear (plastic or glass) indicator wand together with an optical sensor. The wand can be configured in such a way that the sensor is located on the "back" or "dry" side of the wand and when the opposing or "wet" side of the wand is inserted into a quaternary ammonium compound or solution, the dye on the wet side would change color and that color change would be measurable by the sensor.

In some examples, a sensor may be incorporated directly into a dispenser to verify that adequate sanitizer concentrations were dispensed. If the dispenser includes a sensor, then the sensor could provide feedback to the dispenser such that the dispenser keeps dispensing product until the correct concentration of quat has been dispensed. The dispenser could also be programmed to automatically dispense additional product if the concentration of quat at any time drops below the desired threshold.

In some examples, exemplary dyes include dyes that are pH independent and pH stable where the wavelength change is not sensitive to pH swings of at least 0.5 pH units. The dyes exhibit a first color or wavelength when the concentration of quaternary ammonium compound is above a threshold and a second color or wavelength when the concentration of quaternary ammonium compound is below a threshold. Preferred dyes have been found to be purple where the purple is a true purple and not simply a combination of red and blue dyes. Preferred dyes are anionic. The dye may be water soluble or water insoluble. Exemplary dyes include Acid Violet 148, bromothymol blue, and alkali purple. The name of the dye is not indicative of its properties. For example, bromothymol blue is actually purple and not blue. In an example, the dye is not bromothymol blue. According to the invention the dye is Acid Violet 148, commercially available from KeyColour, which is purple in the presence of quaternary ammonium compound and blue in the absence or reduced presence of quaternary ammonium compound. This dye changes color in real time and is independent of the pH of the antimicrobial solution.

The dyes may be incorporated into a variety of articles such as towels, labels, containers, buckets, trays, sinks, spray bottles, liners for containers, buckets, sinks, or spray bottles, indictor wands or strips, coupons, and test kits. Regarding the label, the dye can be incorporated into the label itself or into the ink that gets printed onto the label. The dye can also be incorporated into the materials that form the article, such as ink, paper, the plastic, ceramic, metal, or glass that forms a container, strip or coupon, or the material that forms a woven or nonwoven fabric. For example, when synthetic textiles are made, the process starts with resin pellets and is then extruded to make fine filaments. The dye can be incorporated into the resin and then extruded to form the filaments. Thus the dye will be integral with the fiber that goes on to form thread and eventually a woven or nonwoven textile. The dye can also be incorporated into a paint and then painted onto the article. Exemplary plastics include high density polyethylene, low density polyethylene, linear low density polyethylene, ethylene vinyl acetate, ethylene methyl acrylate, ethylene acrylic acid, ethylene methacrylic acid, polypropylene, nylon, PET, PVC (polyvinyl chloride), PVdC, EVOH, polyurethane, Barex, polystyrene, and combinations thereof. The concentration of the dye can be increased or decreased to change at a higher or lower concentration of quaternary ammonium compound. A sub-stoichiometric concentration of dye to quaternary ammonium compound has been found to be sufficient for creating the necessary color change.

If the dye is water insoluble, it is not like to leach out into the quaternary ammonium compound solution over time once it is incorporated into an article. But, if the dye is water soluble, it may be desirable to bind the dye to a substrate to prevent leaching of the dye into solution. In some examples, it is desirable to bond the dye to a substrate or matrix in such a way that the dye is bound and prevented from leaching out into solution, but in a way that still allows the bound dye to react with the quat and change wavelength. Exemplary substrates include a white, clear (colorless), or colored paint or lacquer, a polymer or resin for extruding or injection molding plastic articles, a resin, polymer, or natural fiber for forming fabrics.

In some examples, it is advantageous for the color change be passive to the observer, meaning that the user or observer does not need to perform a specific activity such as putting a test strip into the solution in order to see the color change. Examples of this include a color changing container that changes color as the quaternary ammonium concentration changes or using a sensor that continuously monitors and reports the concentration.

The present disclosure may be better understood with reference to the following examples.

## EXAMPLES

### Example 1

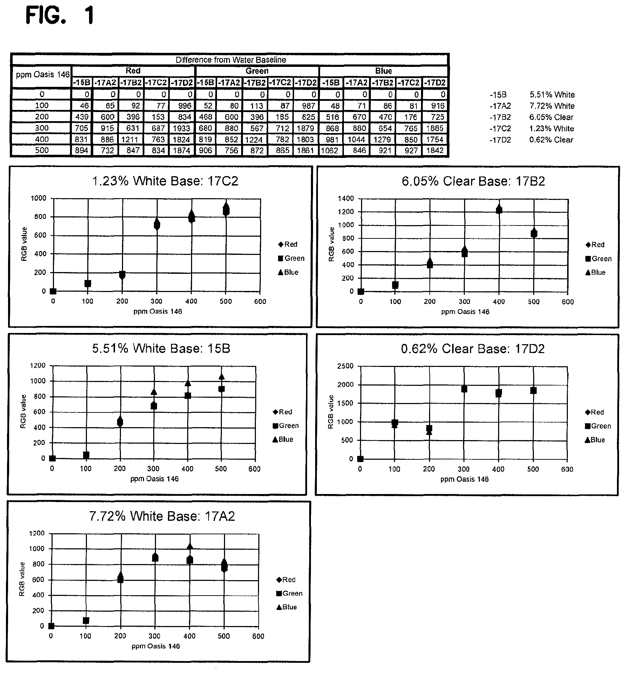

Acid Violet 148 dye (available from Key Colours) was added to both Rust-Oleum 7791 Satin White Enamel and Rust-Oleum 7701 Crystal Clear paints after a quantity of the paint was captured in a bottle by spraying into the bottle. Samples of Satin White contained 1.23, 5.51 and 7.7% w/w Acid Violet 148. Samples of Crystal Clear contained 0.62 and 6.0% w/w Acid Violet 148 in 7701 Crystal Clear. Several drops of quat samples prepared from Oasis 146 (a quaternary ammonium compound antimicrobial composition commercially available from Ecolab Inc.) were placed on the test surface and the color was measured using a TCS230LM programmable color light-to-frequency converter from Texas Advanced Optoelectronic Systems (Piano, Texas). The color measurements from the RGB channels of the color sensor demonstrate a quat concentration dependent response illustrated in Figure 1. Figure 1 shows a quat color test using a 16-bit RGB setting. The individual red, green, and blue values were combined into a single value using the following equation where $R_0$, $G_0$, and $B_0$ are the reference colors and $R_n$, $G_n$, and $B_n$ are the sample measurements:

$$Distance = \sqrt{(R_n - R_0)^2 + (G_n - G_0)^2 + (B_n - B_0)^2}$$

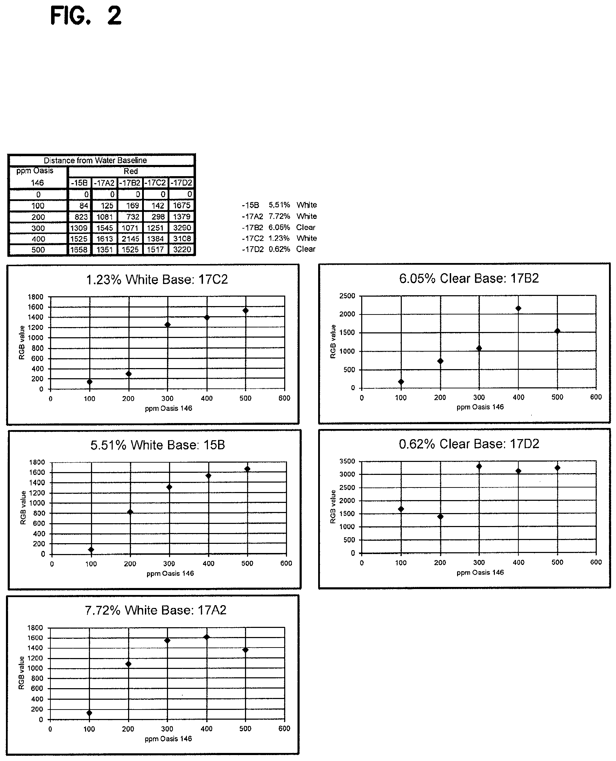

The combined calculations are shown in Figure 2. As the data in Figures 1 and 2 illustrate, the response is also dependent upon the dye concentration in the paint. This suggests that the response can be optimized for the desired quat concentration range to be measured. The response is proportional to the quat concentration. The figures also show the response observed for a clear paint.

The samples according to the invention, with 5.51% dye on white paint, provided the best concentration-dependent response between 150 ppm and 400 ppm of the quat. The slope of the curve at this concentration is smooth and very predictable, which suggests that the changes in the color of dye will be predictable as the concentration of quat changes. This is desirable in the disclosed indicator system. It was also observed that the white paint appeared to be a better substrate (more predictable) than the clear paint.

The above specification, examples and data describe the disclosure. Additional embodiments can be made without departing from the scope of the following claims.

## Claims

1. A system for determining the concentration of a quaternary ammonium compound comprising:

(a) an article selected from the group consisting of a dispenser, a bucket, a sink, a spray bottle, an applicator, a strip, a coupon, a label, a probe, and a sprayer dip tube;
(b) a dye located on or integral with the article, wherein the dye is selected from the group consisting of anionic dyes that are purple in the presence of quaternary ammonium compounds, wherein the dye provides a concentration-dependent response between 150 ppm and 400 ppm of a quaternary ammonium compound; and
(c) a sensor capable of measuring the light intensity of the dye as a function of the wavelength of the dye and converting that measurement to a digital output related to the quaternary ammonium compound concentration, wherein the dye provides a concentration-dependent response between 150 ppm and 400 ppm of the quaternary ammonium compound; and

wherein the dye is Acid Violet 148 with a concentration of 5.51 % incorporated into white paint.

2. The system of claim 1, wherein the dye is integral with the article and the article is selected from the group consisting of a dispenser, a bucket and a sink

3. The system of claim 2, wherein the sensor is incorporated into a wall of the article,

4. The system of claim 1, wherein the article is a label.

5. The system of claim 4, wherein the label comprises:

    (a) a front surface;
    (b) a back surface; and
    (c) an adhesive layer located on either the front surface or back surface.

6. The system of claim 1, wherein the sensor is a handheld pen-sized sensor.

7. The system of claim 1, wherein the sensor is an integrated light-to-frequency converter.

8. The system of claim 7, wherein the sensor includes an RGB color sensor,

9. The system of claim 7, wherein the light-to-frequency provides digital output in real time.

10. A method of monitoring the concentration of a quaternary ammonium compound comprising:

    (a) contacting a quaternary ammonium compound antimicrobial solution having a quaternary ammonium compound concentration greater than 400 ppm with a dye located on or integral with an article, wherein the dye provides a concentration-dependent response between 150 ppm and 400 ppm of a quaternary ammonium compound;
    (b) measuring or deriving the wavelength of the dye with a sensor, wherein the sensor provides an output related to the quaternary ammonium compound concentration;
    (c) using the quaternary ammonium solution for a period of time; and
    (d) adding quaternary ammonium solution in response to the dye changing from a first wavelength to a second wavelength in the presence of between 150 ppm and 400 ppm of a quaternary ammonium compound;

wherein the dye is Acid Violet 148 with a concentration of 5.51 % incorporated into white paint.

11. The method of claim 10, wherein the article is made from a polymer selected from the group consisting of a high density polyethylene, low density polyethylene, linear low density polyethylene, ethylene vinyl acetate, ethylene methyl acrylate, ethylene acrylic acid, ethylene methacrylic acid, polypropylene, polyethylene terephthalate, polyvinyl chloride, polystyrene, and mixtures thereof.

12. The method of claim 10, wherein the sensor is incorporated into a wall of the article.

**Patentansprüche**

1. System zum Messen der Konzentration einer quartären Ammoniumverbindung, umfassend:

    (a) einen Gegenstand, der aus der Gruppe ausgewählt ist, die aus einem Spender, einem Eimer, einem Spülbecken, einer Sprühflasche, einem Applikator, einem Streifen, einem Coupon, einem Etikett, einer Sonde und einem Sprüh-Tauchrohr besteht;
    (b) einen Farbstoff, der sich auf dem Gegenstand befindet oder ein integraler Bestandteil davon ist, wobei der Farbstoff ausgewählt ist aus der Gruppe bestehend aus anionischen Farbstoffen, die in Gegenwart von quartären Ammoniumverbindungen purpurfarben sind, wobei der Farbstoff eine konzentrationsabhängige Reaktion zwischen 150 ppm und 400 ppm einer quartären Ammoniumverbindung liefert; und

(c) einen Sensor, der in der Lage ist, die Lichtintensität des Farbstoffs als Funktion der Wellenlänge des Farbstoffs zu messen und diese Messung in eine digitale Ausgabe umzuwandeln, die sich auf die Konzentration der quartären Ammoniumverbindung bezieht, wobei der Farbstoff eine konzentrationsabhängige Reaktion zwischen 150 ppm und 400 ppm der quartären Ammoniumverbindung liefert; und

wobei der Farbstoff Acid Violet 148 mit einer Konzentration von 5,51 % ist, der in weiße Farbe eingearbeitet ist.

2. System nach Anspruch 1, wobei der Farbstoff integraler Bestandteil des Gegenstands ist und der Gegenstand aus der Gruppe ausgewählt ist, die aus einem Spender, einem Eimer und einer Spüle besteht.

3. System nach Anspruch 2, wobei der Sensor in eine Wand des Gegenstands eingearbeitet ist.

4. System nach Anspruch 1, wobei der Gegenstand ein Etikett ist.

5. System nach Anspruch 4, wobei das Etikett umfasst:

(a) eine Vorderseite;
(b) eine Rückseite; und
(c) eine Klebeschicht, die sich entweder auf der Vorder- oder Rückseite befindet.

6. System nach Anspruch 1, wobei der Sensor ein Handsensor in Stiftgröße ist.

7. System nach Anspruch 1, wobei der Sensor ein integrierter Licht-Frequenz-Wandler ist.

8. System nach Anspruch 7, wobei der Sensor einen RGB-Farbsensor beinhaltet.

9. System nach Anspruch 7, wobei der Licht-Frequenz-Wandler einen Digitalausgang in Echtzeit zur Verfügung stellt.

10. Verfahren zur Überwachung der Konzentration einer quartären Ammoniumverbindung, umfassend

(a) Inkontaktbringen einer antimikrobiellen quartären Ammoniumverbindungslösung mit einer Konzentration der quartären Ammoniumverbindung von mehr als 400 ppm mit einem Farbstoff, der sich auf einem Gegenstand befindet oder integraler Bestandteil davon ist, wobei der Farbstoff eine konzentrationsabhängige Reaktion zwischen 150 ppm und 400 ppm einer quartären Ammoniumverbindung liefert;
(b) Messen oder Ableiten der Wellenlänge des Farbstoffs mit einem Sensor, wobei der Sensor ein mit der Konzentration der quartären Ammoniumverbindung zusammenhängendes Ergebnis liefert;
(c) Verwenden der quartären Ammoniumlösung über einen bestimmten Zeitraum und
(d) Zugabe von quartärer Ammoniumlösung als Reaktion darauf, dass sich der Farbstoff in Gegenwart von 150 ppm bis 400 ppm einer quartären Ammoniumverbindung von einer ersten Wellenlänge zu einer zweiten Wellenlänge ändert;

wobei der Farbstoff Acid Violet 148 mit einer Konzentration von 5,51 % ist, der in weiße Farbe eingearbeitet ist.

11. Verfahren nach Anspruch 10, wobei der Gegenstand aus einem Polymer hergestellt ist, das aus der Gruppe ausgewählt ist, die aus einem hochdichten Polyethylen, niederdichten Polyethylen, linearen niederdichten Polyethylen, Ethylenvinylacetat, Ethylenmethylacrylat, Ethylenacrylsäure, Ethylenmethacrylsäure, Polypropylen, Polyethylenterephthalat, Polyvinylchlorid, Polystyrol und Gemischen davon besteht.

12. Verfahren nach Anspruch 10, wobei der Sensor in eine Wand des Gegenstands eingearbeitet ist.


**Revendications**

1. Système destiné à déterminer la concentration d'un composé d'ammonium quaternaire comprenant:

(a) un article sélectionné dans le groupe constitué d'un distributeur, d'un seau, d'un évier, d'un flacon pulvérisateur, d'un applicateur, d'une bande, d'un coupon, d'une étiquette, d'une sonde et d'un tube plongeur de pulvérisateur ;

(b) un colorant situé sur un article ou faisant partie intégrante de celui-ci, dans lequel le colorant est sélectionné dans le groupe constitué de colorants anioniques qui sont violets en présence de composés d'ammonium quaternaire, dans lequel le colorant fournit une réponse dépendante de la concentration entre 150 ppm et 400 ppm d'un composé d'ammonium quaternaire ; et

(c) un capteur permettant de mesurer l'intensité lumineuse du colorant en fonction de la longueur d'onde du colorant et de convertir cette mesure en une sortie numérique associée à la concentration en composé d'ammonium quaternaire, le colorant fournissant une réponse dépendante de la concentration entre 150 ppm et 400 ppm du composé d'ammonium quaternaire ; et

dans lequel le colorant est de l'acide violet 148 comportant une concentration de 5,51 % incorporée dans la peinture blanche.

2. Système selon la revendication 1, dans lequel le colorant fait partie intégrante de l'article et l'article est sélectionné dans le groupe constitué d'un distributeur, d'un seau et d'un évier.

3. Système selon la revendication 2, dans lequel le capteur est incorporé dans une paroi de l'article.

4. Système selon la revendication 1, dans lequel l'article est une étiquette.

5. Système selon la revendication 4, dans lequel l'étiquette comprend :

(a) une face avant ;
(b) une face arrière ; et
(c) une couche adhésive située sur la face avant ou la face arrière.

6. Système selon la revendication 1, dans lequel le capteur est un capteur de la taille d'un stylo de poche.

7. Système selon la revendication 1, dans lequel le capteur est un convertisseur lumière-fréquence intégré.

8. Système selon la revendication 7, dans lequel le capteur comprend un capteur de couleur RVB.

9. Système selon la revendication 7, dans lequel la lumière-fréquence fournit une sortie numérique en temps réel.

10. Procédé de contrôle de la concentration d'un composé d'ammonium quaternaire comprenant

(a) la mise en contact d'une solution antimicrobienne à composé d'ammonium quaternaire présentant une concentration en composé d'ammonium quaternaire supérieure à 400 ppm comportant un colorant situé sur un article ou faisant partie intégrante de celui-ci, le colorant fournissant une réponse dépendante de la concentration entre 150 ppm et 400 ppm d'un composé d'ammonium quaternaire ;

(b) la mesure ou la dérivation de la longueur d'onde du colorant avec un capteur, dans lequel le capteur fournit un signal de sortie associé à la concentration de composé d'ammonium quaternaire ;

(c) l'utilisation de la solution d'ammonium quaternaire pendant une période temporelle et

(d) l'ajout d'une solution d'ammonium quaternaire en réponse au colorant changeant d'une première longueur d'onde à une seconde longueur d'onde en présence d'entre 150 ppm et 400 ppm d'un composé d'ammonium quaternaire ;

dans lequel le colorant est de l'acide violet 148 comportant une concentration de 5,51 % incorporée dans la peinture blanche.

11. Procédé selon la revendication 10, dans lequel l'article est fabriqué à partir d'un polymère sélectionné dans le groupe constitué d'un polyéthylène haute densité, d'un polyéthylène basse densité, d'un polyéthylène linéaire basse densité, d'éthylène-acétate de vinyle, d'éthylène-méthyle acrylate, d'éthylène-acide acrylique, d'éthylène-acide méthacrylique, de polypropylène, de polyéthylène téréphtalate, de polychlorure de vinyle, de polystyrène et de leurs mélanges.

12. Procédé selon la revendication 10, dans lequel le capteur est incorporé dans une paroi de l'article.

# FIG. 1

| ppm Oasis 146 | Red | | | | | Green | | | | | Blue | | | | |
|---|---|---|---|---|---|---|---|---|---|---|---|---|---|---|---|
| | -15B | -17A2 | -17B2 | -17C2 | -17D2 | -15B | -17A2 | -17B2 | -17C2 | -17D2 | -15B | -17A2 | -17B2 | -17C2 | -17D2 |
| 0 | 0 | 0 | 0 | 0 | 0 | 0 | 0 | 0 | 0 | 0 | 0 | 0 | 0 | 0 | 0 |
| 100 | 46 | 65 | 92 | 77 | 996 | 52 | 80 | 113 | 87 | 987 | 48 | 71 | 86 | 81 | 916 |
| 200 | 439 | 600 | 396 | 153 | 834 | 468 | 600 | 396 | 185 | 825 | 516 | 670 | 470 | 176 | 725 |
| 300 | 705 | 915 | 631 | 687 | 1933 | 680 | 880 | 567 | 712 | 1879 | 868 | 880 | 654 | 765 | 1885 |
| 400 | 831 | 886 | 1211 | 763 | 1824 | 819 | 852 | 1224 | 782 | 1803 | 981 | 1044 | 1279 | 850 | 1754 |
| 500 | 894 | 732 | 847 | 834 | 1874 | 906 | 756 | 872 | 865 | 1861 | 1062 | 846 | 921 | 927 | 1842 |

Difference from Water Baseline

-15B    5.51% White
-17A2   7.72% White
-17B2   6.05% Clear
-17C2   1.23% White
-17D2   0.62% Clear

# FIG. 2

| Distance from Water Baseline | | | | | |
|---|---|---|---|---|---|
| ppm Oasis | Red | | | | |
| 146 | -15B | -17A2 | -17B2 | -17C2 | -17D2 |
| 0 | 0 | 0 | 0 | 0 | 0 |
| 100 | 84 | 125 | 169 | 142 | 1675 |
| 200 | 823 | 1081 | 732 | 298 | 1379 |
| 300 | 1309 | 1545 | 1071 | 1251 | 3290 |
| 400 | 1525 | 1613 | 2145 | 1384 | 3108 |
| 500 | 1658 | 1351 | 1525 | 1517 | 3220 |

-15B   5.51%   White
-17A2  7.72%   White
-17B2  6.05%   Clear
-17C2  1.23%   White
-17D2  0.62%   Clear

1.23% White Base: 17C2

6.05% Clear Base: 17B2

5.51% White Base: 15B

0.62% Clear Base: 17D2

7.72% White Base: 17A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100247371 A1